# EUROPEAN PATENT SPECIFICATION

(11) **EP 1 335 900 B1**
(45) Date of publication and mention of the grant of the patent: **18.04.2007**
(21) Application number: 01990778.1
(22) Date of filing: 20.11.2001
(51) Int. Cl.: C07C 323/52, C07C 323/35, C07C 323/36, C07C 323/37, C07C 323/62, C07D 213/70, C07D 235/28, C07D 239/56, C08K 5/372, C08K 5/38

(54) **ALKYLTHIO- AND ARYL(HETEROYL)THIO-SUBSTITUTED P-PHENYLENEDIAMINES, THEIR MANUFACTURE AND THEIR USE IN RUBBER**
ALKYLTHIO- UND ARYL(HETEROYL)THIO-SUBSTITUIERTE P-PHENYLENDIAMINE, DEREN HERSTELLUNG UND DEREN VERWENDUNG IN KAUTSCHUK
ALKYLTHIO-ET ARYL(HETEROYL)THIO-SUBSTITUES P-PHENYLENEDIAMINES, LEURS FABRICATION ET LEUR UTILISATION DANS LE CAOUTCHOUC

(30) Priority: 21.11.2000 US 252679 P
(43) Date of publication of application: 20.08.2003
(73) Proprietor: FLEXSYS AMERICA L.P., Akron, OH 44334-4111 (US)
(72) Inventor: MAENDER, Otto, William, John's Island, South Carolina 29455 (US); ROSTEK, Charles, J., Chagrin Falls, OH 44022 (US); KATRITZKY, Alan, R., Gainesville, FL 32601 (US); ODENS, Herman, H., Wilmington, DE 19808 (US); VORONKOV, Michael, V., Cranbury, NJ 08512 (US)
(74) Representative: Beetz, Tom
(86) International application number: PCT/US2001/045213
(87) International publication number: WO 2002/042262

(56) References cited:
- EP-A- 0 001 604
- US-A- 2 991 271
- US-A- 4 137 310
- US-A- 5 414 131
- US-A- 5 542 952
- J.D. SCRIBNER, ET AL.: "Intramolecular hydrogen bonding in o-methylmercapto derivatives of N-methylaniline and N-methyl-4-aminoazobenzene" JOURNAL OF ORGANIC CHEMISTRY, vol. 32, no. 7, July 1967 (1967-07), pages 2348-2349, XP002220292 American Chemical Society, Washington, DC, US ISSN: 0022-3263
- T. ZINCKE, ET AL.: "Über 1,4-Aminothiophenol III" BERICHTE DER DEUTSCHEN CHEMISCHEN GESELLSCHAFT, vol. 44, 1911, pages 614-626, XP002220429 Verlag Chemie, Weinheim, DE

## Description

### BACKGROUND OF THE INVENTION

### Field of the Invention

The invention relates to alkylthio- and aryl(heteroyl)thio-substituted p-phenylenediamines, their manufacture and use as antidegradants in rubber compounds.

### Discussion of the Prior Art

Vulcanizing rubber compositions by heating a sulfur-vulcanizable rubber composition with sulfur and/or a sulfur donor and a vulcanization accelerator has been known for many years. By this process vulcanizates having acceptable physical properties including tensile strength, resilience, and fatigue resistance can be obtained, but such vulcanizates tend not to have good aging properties.

Uncured as well as cured rubbers are prone to aging effects. The unsaturated groups in diene rubbers, e.g. styrene-butadiene rubber (SBR) or a blend of SBR with natural rubber, butadiene rubber or with both, make it possible to cure with sulfur, but at the same time they exhibit a sensitivity toward oxygen, ozone, and other reactive substances causing changes such as hardening of the vulcanizate. Unaged diene rubbers contain free double bonds that remain sensitive to the above reactive substances even after vulcanization. Higher temperatures make these effects even more noticeable.

Protective agents are used to protect the rubber vulcanizate against various forms of aging, fatigue, and ozone. For example, exposure of pneumatic tires to ozone leads to the formation of ozone cracks, in particular in the sidewalls of the tire. A well-known class of protective agents are N,N'-disubstituted, in particular N-alkyl-N'-phenyl-p-phenylenediamine derivatives. These N,N'-di-substituted p-phenylenediamine derivatives typically are also referred to as antidegradants, antiozonants or antioxidants. The reader is directed to Hofmann, Rubber Technology Handbook, Hanser Publishers, Munich 1989, pp. 264-277, in particular pp. 269-270. These antidegradants are commercially available *inter alia* under the trademark Santoflex^{®} sold by Flexsys America L.P. In the rubber industry, the most frequently used antidegradant is N-(1,3-dimethylbutyl)-N'-phenyl-p-phenylenediamine or 6PPD.

Known 1,4-benzoquinone diimines (QDI's) can be classified under three major categories: I, II and III. Class I compounds contain no substituents attached to the benzoquinoid ring. Class II, the largest group, contains amino-substituents at the 2- and 5-positions of the benzoquinoid ring. Class III comprises all other p-benzoquinone diimines.

Many benzoquinone diimines (QDl's) were previously prepared by the oxidation of phenylenediamine derivatives with manganate, ferricyanate, iodine, silver ion, silver oxide, and lead tetraacetate (or "red lead").

Oxidation of N,N'-diphenyl-*p*-phenylenediamine with Ag₂O gives N,N'-diphenyl-p-phenylenediimine. Oxidation of N-(1,3-dimethylbutyl)-N'-phenyl-1,4-phenylenediamine (6PPD), an effective antiozonant and antioxidant used in rubber industry, with Ag₂O gives the corresponding QDI in 55% yield; similar conversion is achieved by photocatalytic oxidation using Ru³. The QDI can be prepared by two consecutive photo cleavages of NO groups from the bis-nitroso derivative of N,N'-diphenyl-p-phenylenediamine.

N,N'-Diaryl-2,5-bis(arylamino)-p-benzoquinone diimines or azophenines, which belong to the second class of QDI's and are dye intermediates, have been synthesized by a wide variety of methods many of which involve the separation of resulting mixtures and afford low yields. Parent azophenine has been prepared by heating p-benzoquinonedianil with aniline. Substituted azophenines are also formed in 25-35% yields by oxidation of anilines on heating with 1,1,2,2-tetrachloroethane or hexachloroethane in the presence of copper bronze. The oxidation of aniline by 3- and 4-azidopyridine-1-oxides also gives azophenines. Peroxidase oxidation of 4-chloroaniline gives 2-amino-5-chloroanilinobenzoquinone di-4-chloroanil. Benzoquinone diimines are formed as side products in the decomposition of the corresponding nitroxide.

N,N'-Bis[phenylsulfonyl]-1,4-benzoquinone diimine is sulfanylamidated by sodium N-chlorobenzenesulfonamide to form 2,5-bis(phenylsulfonylamino)-N,N'-bis(phenylsulfonyl)-1,4-benzoquinone diimine. Similarly, amidation of N,N'-bis[phenylsulfonyl]-1,4-benzoquinone diimine with N-chloroamides gives the corresponding sulfonyl benzoquinone diimine derivatives in one step.

Few synthetic procedures are known for the preparation of representatives of class III of benzoquinone diimines. For example, many symmetrical and unsymmetrical N,N'-bis(arylthio)- and N,N'-bis(arylseleno)-quinone diimines have been prepared by treatment of N,N'-dichloroquinone diimines with thiols or selenols, respectively.

More recently, N,N'-dicyanoquinone diimine (DCNQI) salts have gained attention due to their high conductivity and ease of synthesis from benzoquinones and bis(trimethylsilyl)carbodiimide.

A synthetic route to poly(quinone diimines) *via* the treatment of anthraquinone (AQ) with aromatic diamines in the presence of TiCl₄ and 1,4-diazabicyclo[2.2.2] octane (Dabco) has been developed.

Employing the same model as above, some heterocyclic quinone arylimines have been prepared through Wittig addition of (N-aryl)triphenylarsinimines to the carbonyl functionality of heterocyclic benzoquinone derivatives. Polyaromatic quinone imines are readily formed by reactions of the corresponding quinones with triphenylarsine oxide and aryl isocyanates.

Most reactions of p-benzoquinone diimines fall into two broad types: reduction and addition. The reactivity of benzoquinone diimines (QDI) is dictated by a strong tendency to form a stable benzenoid structure. Therefore, they are very reactive towards nucleophilic addition and undergo reduction more readily than quinones. Most published reactions of QDI's relate to studies of the relatively stable diacyl and disulfonyl derivatives.

In the publication by Snell and Weissberger, *The Reaction of Thiol Compounds with Quinones,* JACS 61, 450(1938), the reactions between thiol compounds with benzoquinone and substituted benzoquinones were discussed. The article stated that two types of reaction may be expected: oxidation of the thiol to a disulfide with reduction of the quinone to the hydroquinone; and addition of the thiol to the quinone to obtain alkylthio substituted quinones and/or hydroquinones.

In Gelling and Knight *Rubber chemistry of N-substituted quinone imines and N, N'-disubstituted quinone diimines,* Plastics and Rubber Processing September 1977, findings were discussed concerning two distinct reactions that occur between 2-mercaptobenzothiazole (MBT) and N-cyclohexyl-N'-phenylquinone diimine. The major reaction involves 1,4-addition of the MBT across the diimine ring to yield the addition product There is also, an oxidation-reduction reaction to yield N-cyclohexyl-N'-phenyl-p-phenylenediamine and 2, 2'-dithio-bis(benzothiazole).

US 2,991,271 discloses reaction products of a sulfur chloride and certain N,N'-dialkyl phenylenediamines for use as antiozonants in rubber.

US 5,542,952 discloses dye compositions for the dyeing of keratin fibers comprising certain sulfur-containing p-phenylenediamines, including 2-methylthio-p-phenylenendiamine and 2-ethylthio-p-phenylenendiamine which latter two compositions as such are not within the scope of protection .

### SUMMARY OF THE INVENTION

In one embodiment the present invention comprises a composition comprising 2-atkylthio- or 2-aryl(heteroyl)thio-substituted p-phenylenediamines having the formula: 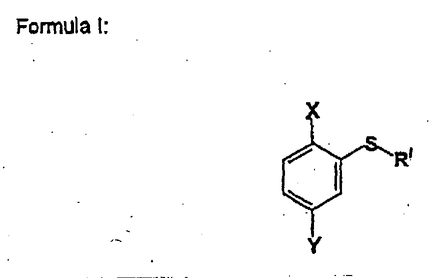 Where:
X and Y are the same or different and selected from the group NH₂, or NHR (where R is H, alkyl, cycloalkyl or aryl) ; and R' is alkyl, cycloalkyl, alkylene, aryl, arylene, alkyl 3-propionate, bridging groups having the formula: -(R"-Z-R")-, where Z is O, NH, NR, S, -SS-, or -(CH₂)ₙCO(R") OC(CH₂)ₙ-, where n=1-3 and R" is not H and is selected from the group consisting of alkylene, arylene, pentaerithrityl and carbon based heterocyclic groups containing at least one of S or N, or both S and N, excluding 2-methylthio-p-phenylenendiamine and 2-ethylthio-p-phenylenendiamine.

In a second embodiment, the present invention comprises a process for the manufacture of the above compositions comprising reacting a quinone diimine and a thiol in accordance with the following reaction equation:

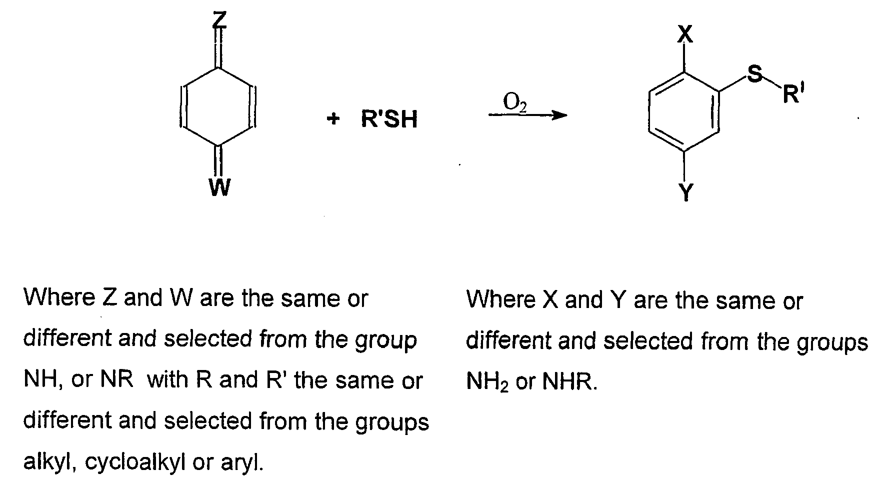

In a third embodiment, the present invention comprises a composition comprising natural or synthetic rubber, or a blend thereof, and one or more antidegradants selected from the above Formula I.

Other embodiments of the invention encompass specific p-phenylenediamines, details concerning their manufacture, and relative amounts of reactants and natural or synthetic rubber compositions, all of which are hereinafter disclosed in the following discussion of each of the facets of the present invention.

### DETAILED DESCRIPTION OF THE INVENTION

Quinonediimines have been described as primary intermediates in the action of p-phenylenediamines as antioxidants and antiozonants. Quinoid structures are highly reactive, undergoing addition reactions by means of either free radicai or polar mechanisms. The formation of 2-alkylthio- and 2,5-bis-amino-1,4-phenylenediamines may be accomplished by the addition of thiols and amines to N-(1,3-dimethylbutyl)-N'-phenyl-1,4-quinonediimine in the presence of air.

According to the present invention, it has been found that by adding appropriate amounts of the above 2-alkylthio- or 2-aryl(heteroyl)thio- substituted p-phenylenediamines of Formula I to a vulcanizable rubber composition comprising natural rubber or other rubbers, vulcanizates, from which, e.g., pneumatic tires can be made, having improved anti-aging, fatigue, and ozone resistance properties, can be obtained.

In this application, the abbreviation "phr" means the number of parts by weight per 100 parts by weight of rubber. In the case of a rubber blend, it is based on 100 parts by weight of total rubber.

Either natural rubber (NR), styrene-butadiene rubber (SBR) or a blend of NR and SBR or NR and SBR with one or more other rubbers can be used in the invention process, it being understood that for purposes of this invention the term "rubber" means an elastomer containing a hydrocarbon unit which is a polymer with some unsaturated chemical bonds. Typically, SBR, a blend of SBR with natural rubber (NR), a blend of SBR with polybutadiene rubber or butadiene rubber (BR), or a blend of SBR with NR and BR is used. The type of rubber or mixture of rubbers will have some effect on the precise amounts of antidegradant to be used.

Typically, the amount of antidegradant employed in the rubber composition of the present invention will be at least about 0.5 phr. The preferred upper limit is about 5.0 phr, most preferably 3.0 phr.

In a preferred embodiment, the composition of Formula I comprises a heteroylthio-substituted p-phenylenediamine wherein R' is a heterocyclic moiety selected from the group consisting of 2-pyrazines, 3-pyrimidines, 2, 3, 4-pyridines, 2-pyrimidines, 2-(4,6-dimethyl) pyrimidines and substituted triazenes.

In another preferred embodiment, the above 2-alkylthio- or 2-aryl(heteroyl)thio-substituted p-phenylenediamine structure of Formula I may comprise a heteroylthio-substituted p-phenylenediamine wherein R' is a heterocyclic moiety selected from the group consisting of 2-pyrazines, 3-pyrimidines, 2-, 3-, 4-pyridines, 2-pyrimidines, 2-(4,6-dimethyl) pyrimidines and substituted triazenes.

Where R' of Formula I is a bridging group, S will be bonded to the bridging group, and the preferred bridging group has the formula: -(R"-Z-R")-, where Z is O, NH, NR, S, -SS-, or -(CH₂)nCO(R") OC(CH₂)n-, where n=1-3 and R" is not H and is selected from the group consisting of alkylene, arylene, pentaerithrityl and carbon-based heterocyclic groups containing at least one of S or N, or both S and N. A highly preferred composition is a heteroylthio-substituted p-phenylenediamine wherein R' is a heterocyclic moiety selected from the group consisting of 1,3,5-triazinyl, 2,5-thiadiazolyl and 2,6-pyridyl.

In another preferred embodiment of Formula I, X is an unsymmetrical p-phenylenediamine having an aminoalkyl group, Y is an amino aryl moiety and the 2-alkylthio- or 2-aryl(heteroyl)thio- group of the substituted p-phenylenediamines is at the 2-position relative to the aminoalkyl group of the unsymmetrical p-phenylenediamine.

In the above embodiment of the present invention for a process for the manufacture of 2-alkylthio- or 2-aryl(heteroyl)thio-substituted p-phenylenediamines, the preferred amount of R'SH employed in the reaction is from 10% to 90% of the stoichiometry required to make a 1:1 adduct, resulting in a reaction product comprising a blend of 2-alkylthio- or 2-aryl(heteroyl)thio-substituted p-phenylenediamines and unreacted quinone diimine.

The most preferred embodiment of the above Formula I of the present invention for a process for the manufacture of 2-alkylthio- or 2-aryl(heteroyl)thio-substituted p-phenylenediamines is in accordance with the following reaction Equation 1 (R' is as defined in Formula I):

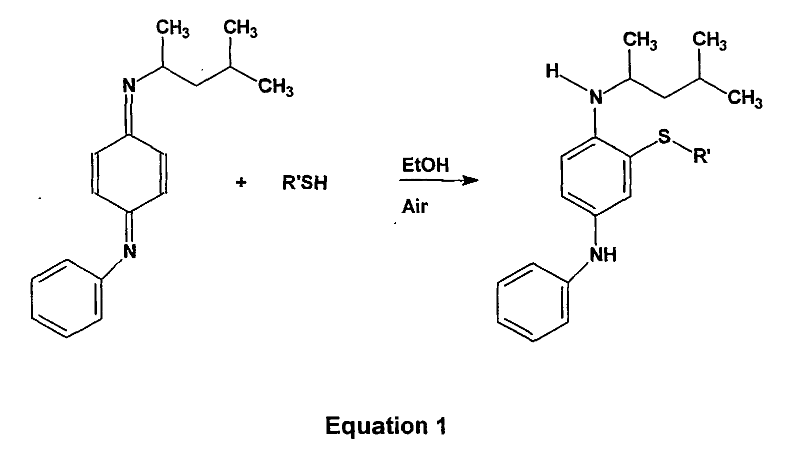

Preferred reaction conditions for the addition reaction of the present invention comprise stirring the reactants dissolved in an appropriate solvent, such as ethanol, for as little as about 2 hours under a constant stream of air from 20° C to 25° C. Surprisingly, the oxidative conditions lead to simple and selective addition of the mercaptan to the quinoidal ring without generating disulfide by-products which can occur under oxidative conditions described in the prior art.

The composition of the present invention and the addition reaction by which it is obtained requires that the alkylthio- or aryl(heteroyl)thio- group is substituted on the aromatic ring at the 2-position next to the alkylamino group of the unsymmetrically substituted p-phenylenediamine. Determination of whether the addition reaction product fulfills that requirement involves two analyses. The first analysis is an elemental analysis of the product that serves to confirm that the desired addition reaction between the particular mercapto derivative and the parent QDI molecule has taken place.

Elemental analysis may be carried out by many methods known to the art, the traditional method comprising burning of the sample and measuring the various products of combustion, such as water, carbon dioxide and free nitrogen.

The second analysis serves to verify placement on the 2-position as stated above. This analysis utilizes nuclear magnetic resonance (NMR) spectroscopy. The specialized NMR techniques of nuclear Overhauser effect (NOE), correlation spectroscopy (COSY) and hetero nuclear correlation (HetCor) also establish the 2-position as the point of attachment.

When 2-alkylthio- or 2-aryl(heteroyl)thio-substituted p-phenylenediamines are prepared by reacting corresponding quinone diimines and thiols in accordance with the process of the present invention, only the single regioisomer is observed in all cases. With reference to the following diagram and accompanying explanation, the assignment of (**27**) rather than the isomeric 3-substituted structure (**30**) is fully justified.

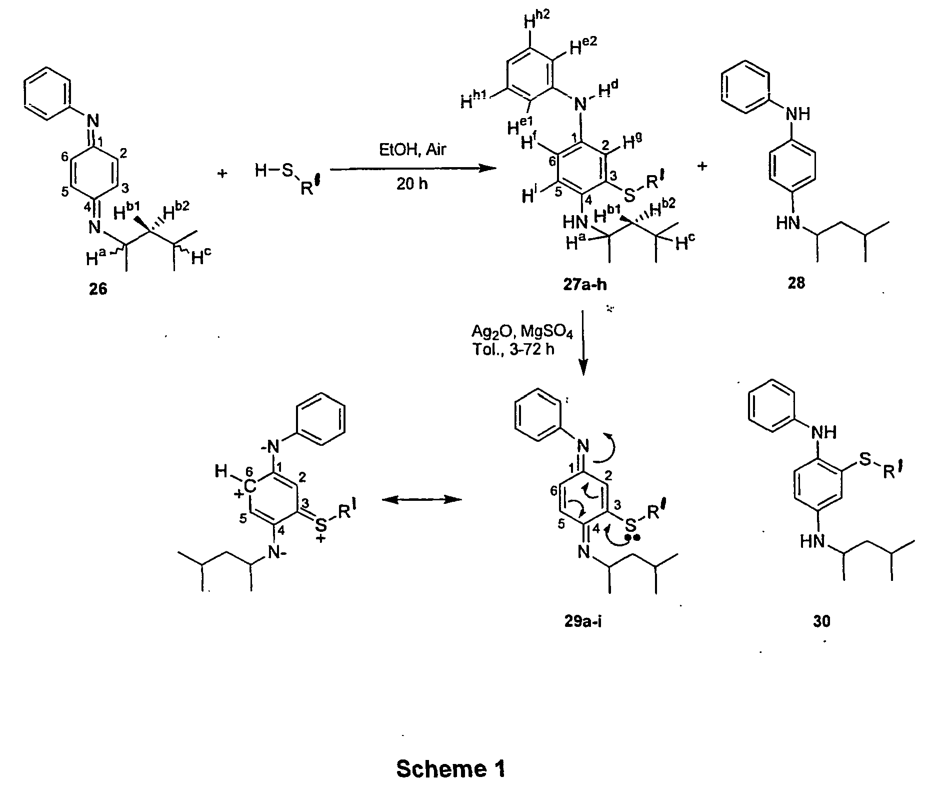

In the ¹H NMR spectrum of **26**, protons H^{b1}, H^{b2}, and H^{c} are shown as a multiplet at 1.67-1.38 ppm. Proton H^{a} signals as a multiplet at 4.07-3.94 ppm (Scheme 1). That the thiol group adds at the *C-3* position of QDI **26** to give compounds **27a-h** is supported by the fact that protons H^{b1}, H^{b2}, and H^{c} split into three separate multiplets at 1.82-1.52 ppm, 1.51-1.36 ppm, and 1.30-1.16 ppm respectively, and the H^{a} proton is a multiplet at 3.54 -3.42 ppm. Due to free rotation around the N-C (4) bond in **27a-h** (in contrast to the rigid E/Z conformation for N=C (4) in compound **26**), the thiol group at *C-3* in the phenylenediamine ring creates a new environment for protons H^{b1}, H^{b2}, and H^{c} resulting in another three different chemical shifts. Besides showing the characteristic ¹³C NMR signals for the quaternary carbons of the two N-C bonds for the phenylenediamine groups at 145.9-142.4 ppm, compounds **27a-h** show the newly formed quaternary carbon at *C-3* with a chemical shift around 128.0-122.1 ppm when an alkyl group is attached to the sulfur atom and 135.7-131.2 ppm for the heterocyclic derivatives respectively.

The composition of the present invention may comprise natural or synthetic rubber or a blend thereof and one or more antidegradants selected from the composition of Formula I. A preferred rubber is polyisoprene. The most preferred antidegradent is the reaction product of Equation I.

The natural or synthetic rubber or blend thereof may comprise a mixture of two or more antidegradants selected from the antidegradants of Formula I or one or more antidegradants selected from the antidegradants of Formula I in combination with a non-thio antidegradant. Preferred non-thio-substituted antidegradants are selected from the group consisting of phenylenediamines, dihydroquinolines and phenolics, or a blend thereof.

It is preferred that the alkyl, cycloalkyl, aryl, arylene and alkylene groups of the composition of the present invention have from 2 to 18 carbon atoms and most preferably 2 to 12 carbon atoms.

A typical rubber composition in accordance with the present invention comprises a rubber, 0.1 to 5 phr of sulfur, 0.5 to 2 phr of a vulcanization accelerator, preferably a sulfenamide accelerator, 0.1 to 5 phr (preferably 2 to 3 phr) of the antidegradant of the invention and a C₁₂-C₂₀ fatty acid such as stearic acid. Metal oxides such as zinc oxide typically are added to rubber compositions.

The rubber composition of the present invention typically also comprises a reinforcing filler in a conventional amount. Any carbon black or combination of carbon black with any silica may be used.

Conventional rubber additives may also be incorporated in the rubber composition according to the present invention. Examples include antireversion agents, processing oils, tackifiers, waxes, phenolic antioxidants, pigments, e.g. titanium dioxide, resins, plasticizers, and factices. These conventional rubber additives may be added in amounts known to the person skilled in the art of rubber compounding. The reader is also referred to the Examples described below.

Conventional rubber additives may also be included in the sulfur-vulcanizable rubber composition in accordance with the present invention. Examples include reinforcing agents such as carbon black, silica, clay, whiting and other mineral fillers, processing oils, tackifiers, waxes, phenolic antioxidants, phenylenediamine antidegradants, antiozonants, pigments, e.g. titanium dioxide, resins, plasticizers, factices, and vulcanization activators, such as stearic acid and zinc oxide. These conventional rubber additives may be added in amounts known to the person skilled in the art of rubber compounding. The reader is also referred to the examples that are described below.

For further details on these typical rubber additives and vulcanization inhibitors, see W. Hofmann, Rubber Technology Handbook, Hanser Publishers, Munich 1989.

Finally, in specific applications it may also be desirable to include steel-cord adhesion promoters such as cobalt salts and bis-thiosulfates in conventional, known quantities.

The composition of the present invention is useful in the manufacture of many articles, including pneumatic tires, e.g., for passenger cars and trucks, and industrial rubber goods, which comprise the rubber vulcanizate obtained by the method of the invention.

The invention is illustrated by the following examples.

### Examples I-15

Each of these examples involved preparation of an N'-(1,3,-dimethylbutyl)-2-(sulfanyl-substituted)-N'-phenyl-1,4-benzenediamines by reacting N'-(1,3,-dimethylbutyl)- N'-phenyl-p-quinone diimine with a mercapto derivative as summarized in Table 1. In each example, the p-quinone diimine (0.80 g, 3.0 mmol) was dissolved in ethanol (25 mL) and treated with the mercapto derivative (0.33 mL, 3.0 mmol) under a constant gentle stream of air. The dark reaction mixture was further stirred for as little as 2 hours and as long as 20 hours over a 20-25 C. temperature range. The solvent was then evaporated and the dark brown slurry was purified by silica gel column chromatography to give the corresponding p-phenylenediamine.

The oxidation induction times (OIT) of each mercapto derivative of examples 1-15 (also given in Table 1) was obtained, thus indicating their respective capacities as antioxidants. In the OIT procedure, a sample of 0.5 wt.% antioxidant in a polymer¹ is used for DSC oxidation induction time analysis.
¹ *cis* Polyisoprene made from synthetic rubber Average *Mw ca.* 40,000 (GPC) from Aldrich 43-126-5.

The sample is run on a TA Instruments 2910 differential scanning calorimeter equipped with nitrogen delivery at 30 ml/min and 100% oxygen delivery at 70 ml/min. An isothermal program is used at 160°C under oxygen until an oxidation exotherm is detected². The sample is first equilibrated at 160°C under nitrogen. Oxygen is then turned on when the isothermal step of the program starts. The oxidation induction time is measured from the point when oxygen is turned on to the onset of the oxidation exotherm.
² The isothermal temperature can be adjusted for an antioxidant/polymer system to give an exotherm which occurs between about 15 min. to an hour.

Oxidation induction times were determined in polyisoprene at 160° C and were also determined for N-(1,3-dimethylbutyl)-N'-phenyl-p-phenylene diamine which exhibited an average OIT of 13.5 minutes under the same conditions as the products of Examples 1-15 (see Table1).

Superior antioxidant capacity is thus shown by the compounds of the present invention as compared to N-1,3-dimethylbutyl-N'-phenyl-p-phenylenediamine. From the OIT data of Table 1, it is observed that the use of the antidegradant of the present invention in polyisoprene in almost every instance is superior to that of N-(1,3-dimethylbutyl)-N'-phenyl-p-phenylene diamine.

**Table 1 - Examples of Thio-substituted-p-phenylenediamines and Their Respective Oxidation induction Times (OIT)**

| Example No. | Mercapto Derivative | OIT Minutes |
|---|---|---|
| **1** | Methyl 3-mercaptopropionate | 88.5 |
| **2** | Cyclohexyl mercaptan | 70.1 |
| **3** | Dodecyl mercaptan | 150.4 |
| **4** | Isopropyl mercaptan | 34.3 |
| **5** | n-Butyl mercaptan | 55.7 |
| **6** | 2-Mercaptopyrimidine | 23.0 |
| **7** | 4,6-Dimethyl-2-mercaptopyrimidine | 6.8 |
| **8** | t-Butyl mercaptan | 24.4 |
| **9** | 2-Mercaptotoluimidazole | 31.2 |
| **10** | Phenyl mercaptan | 60.4 |
| **11** | Benzyl mercaptan | 44.5 |
| **12** | 2-Chlorobenzyl mercaptan | 61.7 |
| **13** | 2-Carboxyphenyl mercaptan | 16.1 |
| **14** | 1,5-Dimercaptoethyl ether | 121.0 |
| **15** | 3-Hydroxy-2-mercaptopyridine | 89.0 |

### Example 16

Each of the thio-substituted-p-phenylenediamines of Examples 1-15 were subjected to an elemental analysis for nitrogen for determination that the desired addition reaction occurred. The analyses for nitrogen is carried out by weighing the sample to be analyzed and subjecting it to quantitative oxidation in a stream of helium and oxygen at temperatures in excess of 1,000 degrees centigrade. After complete combustion, the gasses are passed through a reductor which is packed with copper wire or mesh heated to 650 degrees centigrade. The reductor, by eliminating the excess oxygen, leaves the three combustion gasses (nitrogen, carbon dioxide, and water) ready for injection into the gas chromatograph, and can be considered the interface between the combustion section and the measurement (gas chromatography)section. The gas chromatography section separates the individual components of the gas stream so that they can be individually measured by the detection system. The chromatographic data are digitized, integrated, and mathematically processed to give the elemental composition of the sample.

Table 2 itemizes the yields, physical nature and analyses for nitrogen of the thio-substituted-p-phenylenediamines of Examples 1-15. The nitrogen found as well as the theoretical nitrogen for the structure in question is given. The difference between the two is within expected norms.

**Table 2**

| No. | Mercapto Derivative | Yield % | State -m.p. °C | N, Found (Theory) |
|---|---|---|---|---|
| **1** | Methyl 3-mercaptopropionate | 90 | Yellow oil | 7.53, (7.25) |
| **2** | Cyclohexyl mercaptan | 81 | Yellow oil | 7.22, (7.32) |
| **3** | Dodecyl mercaptan | 93 | Yellow oil | 6.20, (5.98) |
| **4** | Isopropyl mercaptan | 74 | Orange oil | 8.50, (8.18) |
| **5** | n-Butyl mercaptan | 76 | Orange oil | 7.76 (7.86) |
| **6** | 2-Mercaptopyrimidine | 71 | Beige solid 103-106 | 14.37 (14.81) |
| **7** | 4,6-Dimethyl-2-mercaptopyrimidine | 87 | Beige solid 88-91 | 13.83 (13.78) |
| **8** | t-Butyl mercaptan | 85 | Orange oil | 7.72 (7.86) |
| **9** | 2-Mercaptotoluimidazole | 50 | Yellow solid 73-75 | |
| **10** | Phenyl mercaptan | 75 | Amber oil | |
| **11** | Benzyl mercaptan | 89 | Brown oil | 7.46, (7.17) |
| **12** | 2-Chlorobenzyl mercaptan | 88 | Brown oil | 6.74, (6.59) |
| **13** | 2-Carboxyphenyl mercaptan | 80 | Yellow solid 119-121 | 6.66, (6.64) |
| **14** | 1,5-Dimercaptoethyl ether | 93 | Brown tar | |
| **15** | 3-Hydroxy-2-mercaptopyridine | 65 | Black oil | |

### Example 17

The product of each of Examples 1-15 were further subjected to NMR analysis to provide spectroscopic evidence for selective regioisomer formation, particularly for confirmation that the mercapto derivative becomes attached to the 2-position of the substituted p-phenylenediamine. With reference to Scheme 1, the proton and ¹³C NMR signals were consistent with substitution in the 2-position of the product which is identical to the 3-position of the starting quinone diimine.

NOE experiments were performed using the 3-mercaptopropionate adduct of Example 1 (**see 27a in Scheme 1**) as a typical example. When the anilino NH^{d} proton was irradiated, it produced NOE effects on protons H^{e1} (8.2%), H^{e2} (8.2%), H^{f} (7.1%) and H⁹ (7.1%) (Refer to isomers). Similarly, irradiation of proton H^{e1}, H^{e2} produced NOE effects on protons H^{d} (5.0%), H^{h1} (8.1 %) and H^{h2} (8.1 %) respectively. COSY and HetCor experiments also support the structural configuration of the compound **27a.** There is correlation between the H^{g} proton (shown in the ¹H NMR as a doublet) and the H^{f} proton (shown in the ¹H NMR as a doublet of doublets). COSY and HetCor Analysis of the compound **27a** also disclose a correlation between protons H^{f} and H'. These results also support our proposed structure for the compound **27a.** Thus, addition of the thiol nucleophile occurs at the C-3 position affording compounds **27a-h** rather than **30.**

The elemental nitrogen analysis as well as the NMR analysis resulted in the determination that the structures of the thio-substituted-p-phenylenediamines of Examples 1-15 are as set forth in Table 3.

**Table 3 - Thio-substituted-p-phenylenediamines**

| **Example #** | **Compound** | **Example#** | **Compound** |
|---|---|---|---|
| **1** | 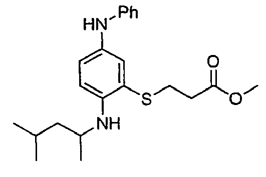 | **5** | 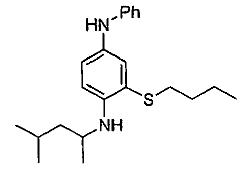 |
| **2** | 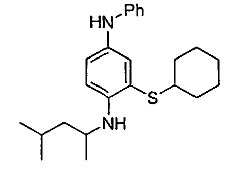 | **6** | 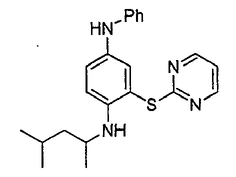 |
| **3** | 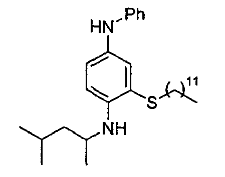 | **7** | 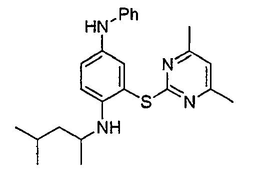 |
| **4** | 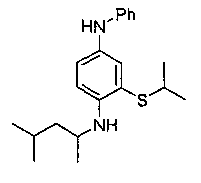 | **8** | 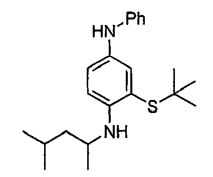 |
| **9** | 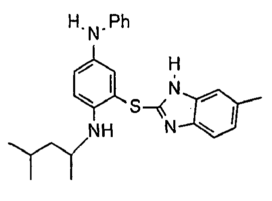 | **13** | 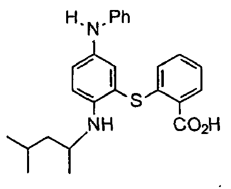 |
| **10** | 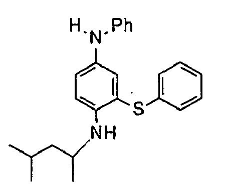 | **14** | 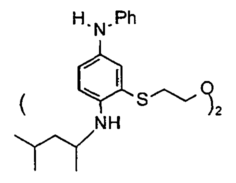 |
| **11** | 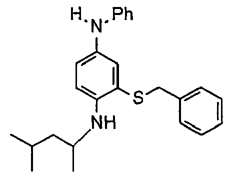 | **15** | 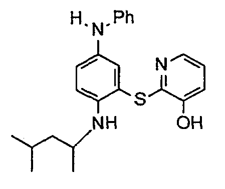 |
| **12** | 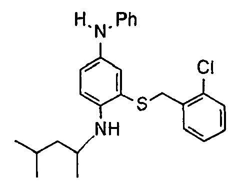 | | |

### Example 18

A masterbatch of rubber, carbon black, lubricant/softener (aromatic oil), and antidegradant was made in an internal mixer. The sulfur, accelerator, and antidegradant were mixed on a two-roll mill at approximately 70-80°C.

Cure characteristics were determined using a Monsanto rheometer ODR 2000E (range 0-100 dNm/arc 1.0°, ASTM D2084-93). Delta torque (Delta S) is the maximum torque (M_{H}) minus the minimum torque (M_{L}). Scorch time or scorch safety (tₛ2) is the time at 2% increase of the minimum torque (M_{L}). Optimum vulcanization or cure time (t₉₀ + 5 min.) is the time required to achieve maximum torque (M_{H}).

Rubber compounds were vulcanized by compression molding at 150°C for t₉₀. After cooling the vulcanized rubber sheets for 24 h, test pieces were cut and analyzed.

Tensile measurements were carried out using a Alpha Technologies T-1 0 tensile tester (ASTM D412 - C dumbbell).

The tensile stress-strain properties were determined in accordance with ASTM D412, the tear strength was determined in accordance with ASTM D624-91, and the fatigue to failure (0-100% extension) in accordance with ASTM 4482/85.

The ozone resistance was tested by comparing the antiozonant capacity of experimental compounds in rubber to that of commercial antidegradants, e.g. Santoflex 6PPD, and unprotected rubber. Stress-strain sheets comprising a rubber formulation that can contain an antidegradant composition are cured in a mold for a time equivalent to that required to achieve a rheometer torque optimum at 150 C. Ozone test specimens are cut from these uniform stress-strain sheets, using a T-50 die (ASTM D2137 with dimensions of 2.54mm width by 50.8mm length with a 6.35mm square at each end to fasten the rubber specimen for testing). These labeled samples are stretched in a standard tensile test machine to obtain the force required to stretch 100% (F(subscript: o)). The test specimens are then exposed to an ozone concentration of 25 pphm at 40° C. in an ozone chamber for periods of 16 hours.

During ozone exposure, the specimens are stretched in racks to a constant 25% extension (static) and are also inserted between two movable disks that allow flexing (extension of 25%) of the rubber specimen under intermittent and continuous (dynamic) cycles. Thus, 3 specimens of each rubber compound are tested concurrently; one static, one intermittent, and one dynamic. Both the dynamic and intermittent disks flex the samples 96 times per minute. Samples are flexed by the intermittent disks for 18 minutes every 2 hours while dynamic flexing proceeds continuously. After 16 hours of ozone exposure the specimens are again tested on the tensile machine to obtain force after aging (F(subscript: t)). The retention value, F (subscript: t) /F(subscript: 0) is calculated and the process of exposure and tensile testing is repeated until the retention value falls below 70% for all the specimens or the specimen breaks due to catastrophic crack formation. The hours to failure or 70% retention of initial force are determined via a regression analysis program and serve as a measure of ozone resistance. Unaged and aged (hot air at 100 C. for 24 hrs.) T-50 specimens are tested in this manner to measure persistence of the antiozonant in the vulcanizate.

The rubber test pieces were aged under one of the following conditions to simulate the service life of rubber during use, for example, as a tire. The test specimens were aged in an air circulation oven for 24, 48 and 72 hours at 100°C.

The masterbatches employed in the compositions were compounded as shown in Tables 4 and 7. The various Stocks comprised the compositions as shown in Tables 5 and 8. The respective kinetic and physical properties are shown in Tables 6 and 9.

For the purpose of comparison, the known antidegradants, Santoflex®6PPD³, Flectol®TMQ⁴, and Wingstay®100⁵ were employed to formulate the various control compounds "C".

The terms "Example #1" and "Example #3" as used in the following discussion are intended to mean the products of the above Example #1 and Example #3, respectively.

**Table 4: SBR Masterbatch**

| Ingredients | phr |
|---|---|
| SBR 1500 | 100 |
| N-330 Carbon Black | 50 |
| Flexon 580⁶ | 10 |
| Zinc Oxide | 4 |
| Stearic Acid | 2 |
| | 166 |

**Table 5: SBR Stock Compositions**

| Stock Number | 1 | 2C | 3C | 4C | 5 | 6 |
|---|---|---|---|---|---|---|
| SBR Masterbatch | 166.0 | 166.0 | 166.0 | 166.0 | 166.0 | 166.0 |
| Sulfur | 2.0 | 2.0 | 2.0 | 2.0 | 2.0 | 2.0 |
| Santocure®CBS ⁷ | 1.2 | 1.2 | 1.2 | 1.2 | 1.2 | 1.2 |
| Santoflex®6PPD | --- | 2.0 | --- | --- | --- | --- |
| Flectol®TMQ | -- | -- | 2.0 | --- | --- | --- |
| Wingstay®100 | --- | --- | --- | 2.0 | --- | --- |
| Example # 1 | --- | --- | -- | --- | 2.0 | --- |
| Example #3 | --- | --- | --- | --- | --- | 2.0 |

| | | | | | | |
|---|---|---|---|---|---|---|
| ³ N-(1,3-Dimethylbutyl)-N'-p-phenylenediamine ⁴ 2,2,4-Trimethyl-1,2-dihydroquinoline ⁵ phenyl-orthotolyl-p-phenylenediamine ⁶ Naphthenic petroleum oil, ASTM type 103 ⁷N-Cyclohexyl-2-benzothiazolesulfenamide | | | | | | |

**Table 6: Kinetic and Physical properties in SBR (refer to Table 5 for Stock compositions)**

| Stock Number | 1 | 2C | 3C | 4C | 5 | 6 |
|---|---|---|---|---|---|---|
| Mooney Scorch @ 135 C. | | | | | | |
| Min. Viscosity | 36.1 | 32.7 | 34.4 | 33.6 | 33.3 | 33.3 |
| t 5, Minutes | 29.1 | 24.3 | 29.8 | 25.0 | 27.3 | 28.8 |
| t 35, Minutes | 38.8 | 30.8 | 38.4 | 34.4 | 36.2 | 39.3 |
| Rheometer @ 150 C. | | | | | | |
| Max. Torque, dNm | 43.0 | 41.1 | 40.5 | 41.8 | 41.3 | 39.7 |
| Min. Torque, dNm | 6.0 | 5.5 | 5.7 | 5.7 | 5.8 | 5.7 |
| t2, Minutes | 12.2 | 11.0 | 12.5 | 11.1 | 11.7 | 12.6 |
| t 90, Minutes | 34.7 | 27.8 | 31.6 | 29.8 | 31.1 | 32.7 |
| t 90 - t 2, Minutes | 22.5 | 16.8 | 19.1 | 18.7 | 19.4 | 20.1 |
| Stress-Strain Data (Unaged) | | | | | | |
| Cured (t90+5) Min. @ 150 C. | | | | | | |
| Tensile, MPa | 21.4 | 24.2 | 23.8 | 24.5 | 21.9 | 23.3 |
| 100% Modulus, MPa | 2.4 | 2.2 | 2.2 | 2.3 | 2.2 | 2.1 |
| 300% Modulus, MPa | 12.3 | 11.4 | 11.2 | 11.8 | 11.5 | 10.9 |
| % Elongation | 465 | 557 | 548 | 546 | 496 | 553 |
| Shore "A" Hardness | 61 | 61 | 61 | 61 | 61 | 60 |
| Stress-Strain Data (Aged) | | | | | | |
| Hot Air Aged 48 hrs. @ 100 C. | | | | | | |
| Tensile, MPa | 17.1 | 18.7 | 19.8 | 17.7 | 18.2 | 18.1 |
| 100% Modulus, MPa | 4.8 | 4.4 | 4.8 | 4.5 | 4.5 | 4.5 |
| 200% Modulus, MPa | 12.9 | 11.8 | 12.7 | 12.0 | 12.1 | 12.2 |
| % Elongation | 249 | 300 | 295 | 281 | 282 | 278 |
| Shore "A" Hardness | 64 | 68 | 65 | 70 | 69 | 67 |
| T50 Ozone Test (Unaged) | | | | | | |
| Static (Hrs to 70% Mod. Ret.) | 17 | 214 | 15 | 46 | 30 | 22 |
| Intermittent (Hrs 70% Mod. Ret.) | 35 | 162 | 41 | 75 | 60 | 50 |
| Dynamic (Hrs to 70% Mod. Ret.) | 25 | 116 | 41 | 93 | 72 | 53 |
| T50 Ozone Test (Aged) | | | | | | |
| 24 Hrs. @ 100 C. | | | | | | |
| Static (Hrs to 70% Mod. Ret.) | 17 | 32 | 18 | 32 | 27 | 24 |
| Intermittent (Hrs 70% Mod. Ret.) | 24 | 61 | 31 | 62 | 42 | 44 |
| Dynamic (Hrs to 70% Mod. Ret.) | 31 | 64 | 48 | 87 | 67 | 53 |

**Table 7: NR Masterbatch**

| Ingredients | phr |
|---|---|
| SMR-CV 60⁸ | 100 |
| N-330 Carbon Black | 50 |
| Flexon 580 | 5 |
| Zinc Oxide | 5 |
| Stearic Acid | 2 |
| | 162 |

**Table 8: NR Stock Compositions**

| Stock Number | 1 | 2C | 3C | 4C | 5 | 6 |
|---|---|---|---|---|---|---|
| NR Masterbatch | 162.0 | 162.0 | 162.0 | 162.0 | 162.0 | 162.0 |
| Sulfur | 2.0 | 2.0 | 2.0 | 2.0 | 2.0 | 2.0 |
| Santocure®CBS | 0.8 | 0.8 | 0.8 | 0.8 | 0.8 | 0.8 |
| Santoflex®6PPD | --- | 2.0 | --- | --- | --- | --- |
| Flectol®TMQ | --- | --- | 2.0 | --- | --- | --- |
| Wingstay®100 | --- | --- | --- | 2.0 | --- | --- |
| Example #1 | --- | --- | --- | --- | 2.0 | --- |
| Example #3 | --- | --- | --- | --- | --- | 2.0 |

| | | | | | | |
|---|---|---|---|---|---|---|
| ⁸ Viscosity-stabilized (Mooney viscosity level 60), high quality grade of natural rubber. | | | | | | |

**Table 9: Kinetic and Physical properties in NR (refer to Table 8 for Stock compositions)**

| Stock Number | 1 | 2C | 3C | 4C | 5 | 6 |
|---|---|---|---|---|---|---|
| Mooney Scorch @ 135 C. | | | | | | |
| Min. Viscosity | 16.3 | 14.9 | 14.9 | 15.3 | 15.2 | 14.5 |
| t 5, Minutes | 14.2 | 13.5 | 14.6 | 13.1 | 13.5 | 15.0 |
| t 35, Minutes | 15.7 | 15.1 | 16.3 | 14.8 | 15.3 | 16.9 |
| Rheometer @ 150 C. | | | | | | |
| Max. Torque, dNm | 33.9 | 33.3 | 32.8 | 34.2 | 33.0 | 32.1 |
| Min. Torque, dNm | 2.4 | 2.3 | 2.2 | 2.4 | 2.3 | 2.1 |
| t 2, Minutes | 5.9 | 5.8 | 6.0 | 5.7 | 5.6 | 6.3 |
| t 90, Minutes | 11.4 | 11.1 | 11.2 | 10.9 | 11.0 | 11.8 |
| t 90 - t 2, Minutes | 5.5 | 5.3 | 5.2 | 5.2 | 5.4 | 5.5 |
| % Reversion | 13.9 | 17.2 | 16.5 | 18.0 | 17.0 | 18.3 |
| Stress-Strain Data (Unaqed) | | | | | | |
| Cured (t90+5) Min. @ 150 C. | | | | | | |
| Tensile, MPa | 26.6 | 26.2 | 27.2 | 27.4 | 26.6 | 25.3 |
| 100% Modulus, MPa | 2.3 | 2.5 | 2.6 | 2.6 | 2.5 | 2.5 |
| 300% Modulus, MPa | 12.4 | 11.6 | 12.4 | 12.3 | 12.0 | 11.8 |
| % Elongation | 541 | 567 | 565 | 570 | 560 | 548 |
| Shore "A" Hardness | 61 | 59 | 60 | 61 | 59 | 58 |
| Stress-Strain Data (Aqed) | | | | | | |
| Hot Air Aged 72 hrs. @ 100 C. | | | | | | |
| Tensile, MPa | 8.7 | 19.9 | 21.6 | 17.0 | 20.0 | 18.5 |
| 100% Modulus, MPa | 2.8 | 4.2 | 4.4 | 4.4 | 4.0 | 4.0 |
| 300% Modulus, MPa | 0.0 | 16.2 | 17.1 | 16.3 | 15.6 | 15.5 |
| % Elongation | 244 | 370 | 383 | 314 | 390 | 361 |
| Shore "A" Hardness | 58 | 64 | 65 | 68 | 66 | 61 |
| Fatigue D4482-85 (Unaqed) | | | | | | |
| Kilocycles to Failure (#8 Cam) | 51 | 293 | 125 | 212 | 99 | 72 |
| Fatigue (Aged 7 days @ 70C.) | | | | | | |
| Kilocycles to Failure (#8 Cam) | 43 | 130 | 86 | 136 | 134 | 71 |
| Die C Tear | | | | | | |
| (ASTM D624-91)21 C. | | | | | | |
| Peak Stress, N/mm | 99.3 | 100.4 | 108.8 | 115.2 | 113.0 | 102.1 |
| Strain, % | 739 | 778 | 825 | 858 | 854 | 808 |
| Die C Tear | | | | | | |
| (ASTM D624-91) 100 C. | | | | | | |
| Peak Stress, N/mm | 64.4 | 60.4 | 62.0 | 65.1 | 67.0 | 66.6 |
| Strain, % | 830 | 738 | 799 | 795 | 882 | 903 |
| T50 Ozone Test (Unaged) | | | | | | |
| Static (Hrs to 70% Mod. Ret.) | 20 | 65 | 19 | 48 | 46 | 42 |
| Intermittent (Hrs 70% Mod. Ret.) | 26 | 90 | 35 | 62 | 59 | 48 |
| Dynamic (Hrs to 70% Mod. Ret.) | 15 | 77 | 32 | 70 | 55 | 49 |
| T50 Ozone Test (Aqed) | | | | | | |
| 24 Hrs. @ 100 C. | | | | | | |
| Static (Hrs to 70% Mod. Ret.) | 20 | 31 | 24 | 44 | 46 | 40 |
| Intermittent (Hrs 70% Mod. Ret.) | 21 | 28 | 22 | 53 | 53 | 48 |
| Dynamic (Hrs to 70% Mod. Ret.) | 16 | 26 | 27 | 50 | 52 | 37 |

With reference to the SBR compositions, Table 5, Examples #1 and #3 were compared to Santoflex®6PPD, Flectol®TMQ, and Wingstay®100 on an equal weight basis. The higher molecular weight of Examples #1 and #3 means that, compared to the control Stocks (2-4C), there are less molar equivalents of antidegradant available to protect the rubber against deterioration. Despite this handicap, Examples #1 and #3 show competitive oxidative capacity after air aging for 48 hrs. at 100 C. (see Table 5). Surprisingly, Examples #1 and #3 exhibit a persistent antiozonant activity, particularly after aging under demanding dynamic conditions as shown in Stocks 5 and 6. While initial antiozonant activity by Examples #1 and #3 is moderate, it is maintained after aging. The comparison of unaged to aged dynamic ozone performance suggests that the higher molecular weight Examples #1 and #3 diffuse to the surface of rubber more slowly than 6PPD, but at a rate sufficient to promote longer-term protection against ozone attack.

The NR formulation and compositions evaluated are shown in Tables 7 and 8. Again, the comparison of Examples #1 and #3 to Santoflex®6PPD, Flectol®TMQ, and Wingstay®100 is made on an equal weight basis. As an indication of antioxidant capacity in compounded NR, Example #1 (Stock 5) shows the best retention of elongation, 70%, after aging and Example #3 (Stock 6) is competitive with the control stocks, 2C and 4C, in retaining elongation (see Table 9). Also in Table 9, Stocks 5 and 6 show good fatigue and tear strength properties, indicating a better preservation of the polysulfide crosslink network. This crosslink stabilization is further supported by better hot tear strength (100 C.) and elongation (strain, %) exhibited by both Examples #1 and #3 in Stocks 5 and 6, respectively.

Moreover, Examples #1 and #3 also show good retention of modulus during aged ozone testing with a clear advantage in aged antiozonant activity over Santoflex®6PPD in NR; compare the T50 Ozone test results of Stocks 5 and 6 to Stock 2 in Table 8.

Surprisingly, in both SBR and NR, Examples #1 and #3 show broad antidegradant activity that favors long-term protection of vulcanizates that may be attributed to molecular size, molecular shape, and a dual mode of action, e.g. chain-stopper and peroxide decomposition, in rubber.

## Claims

1. A composition comprising 2-alkylthio-or 2-aryl (heteroyl)thio-substituted p phenylenediamines having the formula: 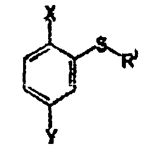 Where:
X and Y are the same or different and selected from the group NH₂, or NHR (where R is H, alkyl, cycloalkyl or aryl) ; and R' is alkyl, cycloalkyl, alkylene, aryl, arylene, alkyl 3-propionate, bridging groups having the formula: -(R"-Z-R")-, where Z is O, NH, NR, S, -SS-,or -(CH₂)ₙCO(R") OC(CH₂)ₙ-, where n=1-3 and R" is not H and is selected from the group consisting of alkylene, arylene, pentaerithrityl and carbon based heterocyclic groups containing at least one of S or N, or both S and N, excluding 2-methylthio-p-phenylenendiamine and 2-ethylthio-p-phenylenendiamine.

2. The composition of claim 1 comprising a heteroylthio-substituted p phenylenediamine wherein R' is a heterocyclic moiety selected from the group consisting of 2-pyrazines, 3-pyrimidines, 2,3,4-pyridines, 2 pyrimidines, 2-(4, 6-dimethyl) pyrimidines and substituted triazenes.

3. The composition of claim 1 comprising a heteroylthio-substituted p phenylenediamine wherein R' is a heterocyclic moiety selected from the group consisting of 1,3,5-triazinyl, 2,5-thiadiazolyl and 2,6-pyridyl.

4. The composition of claim 1 wherein X is an unsymmetrical p phenylenediamine having an aminoalkyl group, Y is an amino aryl moiety and the 2-alkylthio-or 2-, aryl (heteroyl) thio-substituted p-phenylenediamines group is at the 2-position relative to said aminoalkyl group of said unsymmetrical p-phenylenediamine.

5. The composition of claim 1 wherein the alkyl, cycloalkyl, aryl, arylene and alkylene groups have from 2 to 18 carbon atoms.

6. The composition of claim 1 wherein the alkyl, cycloalkyl, aryl, arylene and alkylene groups have from 2 to 12 carbon atoms.

7. A process for the manufacture of 2-alkylthio-or 2-aryl(heteroyl)thio-substituted p-phenylenediamines of claim 1 or of 2-methylthio-p-phenylenendiamine, or 2-ethylthio-p-phenytensndiarnine comprising reacting a quinone diimine and a thiol in accordance with the following reaction equation: 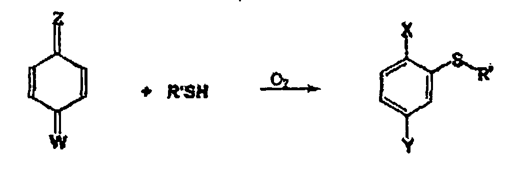 Where Z and W are the same or different and selected from the group NH, or NR with R and R' the same or different and selected from the groups alkyl, cycloalkyl or aryl;
Where X and Y are the same or different and selected from the groups NH₂ or NHR.

8. The process of claim 7 wherein the amount of R'SH employed in the reaction is from 10% to 90% of the stoichiometry required to make a 1: 1 adduct, resulting in a reaction product comprising a blend of 2-alkylthio-or 2 aryl(heteroyl) thio-substituted p-phenylenediamines and unreacted quinone diimine.

9. The process of claim 7 wherein the reaction is in accordance with the following reaction equation:

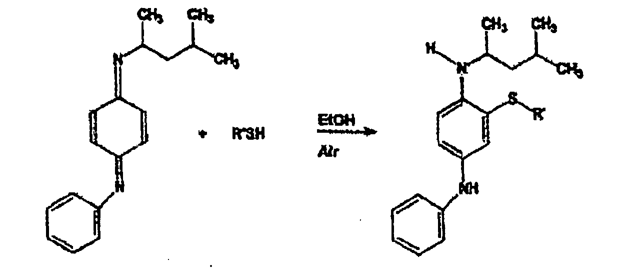

10. The process of claim 7 wherein the reaction conditions comprise stirring the reactants dissolved in an appropriate solvent for at least 2 hours under a constant stream of air at a temperature of from 20° C to 25° C.

11. The process of claim 10 wherein said solvent comprises ethanol.

12. A composition comprising natural or synthetic rubber or blend thereof and one or more antidegradants selected from the composition of claim 1, 2-methylthio-p-phenylenendiamine and 2-ethylthio-p-phenylenendiamine.

13. A composition comprising natural or synthetic rubber or blend thereof and the reaction product of claim 7.

14. The composition of claim 12 wherein the amount of antidegradants employed in the rubber composition is from 0.5 phr. to 5.0 phr.

15. A composition comprising natural or synthetic rubber or blend thereof and a mixture of two or more antidegradants selected from the antidegradants of claim 1, 2-methylthio-p-phenylenendiamine and 2-ethylthio-p-phenylenendiamine, or one or more antidegradants selected from the antidegradants of claim 1, 2-methylthio-p-phenylenendiamine and 2-ethylthio-p-phenylenendiamine in combination with a non-thio antidegradant.

16. The composition of claim 15 wherein said non-thio-substituted antidegradant is selected from the group consisting of phenylenediamines, dihydroquinolines and phenolics, or a blend thereof.

17. The composition of claim 12 wherein said rubber is polyisoprene.

18. The composition of claim 12 comprising from 0.1 phr to 5 phr of sulfur, 0.5 phr to 2 phr of a vulcanization accelerator, 0.1 phr to 5 phr of said antidegradants and a C₁₂-C₂₀ fatty acid.

19. The composition of claim 18 wherein said accelerator is a sulfenamide.

20. The composition of claim 18 comprising from 2 phr to 3 phr of said antidegradants.

## Patentansprüche

1. Zusammensetzung, umfassend 2-alkylthio- oder 2-aryl(heteroyl)thiosubstituierte p-Phenylendiamine mit der Formel 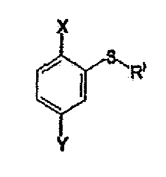 wobei :
X und Y gleich oder verschieden sind und aus der Gruppe NH₂ oder NHR (wobei R H, ein Alkyl, Cycloalkyl oder Aryl ist) ausgewählt sind und R' ein Alkyl, Cycloalkyl, Alkylen, Aryl, Arylen, Alkyl-3-propionat, verbrückende Gruppen mit der Formel: -(R"-Z-R")-, wobei Z O, NH, NR, S, -SS- oder -(CH₂)ₙCO(R")OC(CH₂)ₙ- ist, wobei n = 1 - 3 und R" nicht H und aus der Gruppe ausgewählt ist bestehend aus einem Alkylen, Arylen, Pentaertythrityl und heterocyclischen Gruppen auf der Grundlage von Kohlenstoff, die wenigstens entweder S oder N oder sowohl S als auch N enthalten, wobei 2-Methylthio-p-phenylendiamin und 2-Ethylthio-p-phenylendiamin ausgeschlossen sind.

2. Zusammensetzung nach Anspruch 1, umfassend ein heteroylthiosubstituiertes p-Phenylendiamin, wobei R' ein heterocyclischer Rest ist, der aus der aus 2-Pyrazinen, 3-Pyrimidinen, 2,3,4-Pyridinen, 2-Pyrimidinen, 2-(4,6-Dimethyl)pyrimidinen und substituierten Triazenen bestehenden Gruppe ausgewählt ist.

3. Zusammensetzung nach Anspruch 1, umfassend ein heteroylthiosubstituiertes p-Phenylendiamin, wobei R' ein heterocyclischer Rest ist, der aus der aus 1,3,5-Triazinyl, 2,5-Thiadiazolyl und 2,6-Pyridyl bestehenden Gruppe ausgewählt ist.

4. Zusammensetzung nach Anspruch 1, wobei X ein unsymmetrisches p-Phenylendiamin mit einer Aminoalkylgruppe ist, Y ein Aminoarylrest ist und die 2-alkylthio- oder 2-aryl(heteroyl)thio-substituierte p-Phenylendiamingruppe sich an Position 2 in Bezug auf die Aminoalkylgruppe des unsymmetrischen p-Phenylendiamins befindet.

5. Zusammensetzung nach Anspruch 1, wobei die Alkyl-, Cycloalkyl-, Aryl-, Arylen- und Alkylengruppen 2 bis 18 Kohlenstoffatome haben.

6. Zusammensetzung nach Anspruch 1, wobei die Alkyl-, Cycloalkyl-, Aryl-, Arylen- und Alkylengruppen 2 bis 12 Kohlenstoffatome haben.

7. Verfahren zur Herstellung von 2-alkylthio- oder 2-aryl(heteroyl)thio-substituierten p-Phenylendiaminen nach Anspruch 1 oder 2-Methylthio-p-phenylendiamin oder 2-Ethylthio-p-phenylendiamin, umfassend die Umsetzung eines Chinondiimins und eines Thiols gemäß der folgenden Reaktionsgleichung: 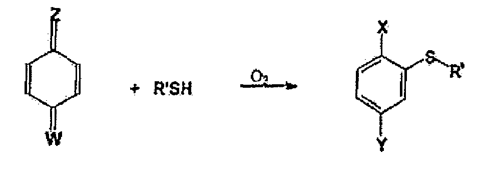 wobei Z und W gleich oder verschieden sind und aus der Gruppe NH oder NR ausgewählt sind, wobei R und R' gleich oder verschieden sind und aus den Gruppen Alkyl, Cycloalkyl oder Aryl ausgewählt sind,
wobei X und Y gleich oder verschieden sind und aus den Gruppen NH₂ oder NHR ausgewählt sind.

8. Verfahren nach Anspruch 7, wobei die in der Reaktion verwendete Menge an R'SH 10 % bis 90 % der Stöchiometrie beträgt, die erforderlich ist, um ein 1:1-Addukt herzustellen, was zu einem Reaktionsprodukt führt, das ein Gemisch aus 2-alkylthio- oder 2-aryl(heteroyl)thio-substituierten p-Phenylendiaminen und nicht umgesetztem Chinondiimin umfasst.

9. Verfahren nach Anspruch 7, wobei die Reaktion gemäß der folgenden Reaktionsgleichung erfolgt:

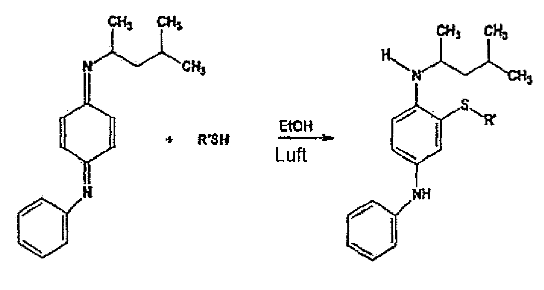

10. Verfahren nach Anspruch 7, wobei die Reaktionsbedingungen das Rühren der in einem zweckmäßigen Lösungsmittel gelösten Reaktanten wenigstens 2 h lang unter einem konstanten Luftstrom bei einer Temperatur von 20 °C bis 25 °C umfasst.

11. Verfahren nach Anspruch 10, wobei das Lösungsmittel Ethanol umfasst.

12. Zusammensetzung, umfassend natürlichen oder synthetischen Kautschuk oder ein Blend davon und eine oder mehrere Zersetzungsschutzmittel, die aus der Zusammensetzung nach Anspruch 1, 1-Methylthio-p-phenylendiamin und 2-Ethylthio-p-phenylendiamin ausgewählt sind.

13. Zusammensetzung, umfassend natürlichen oder synthetischen Kautschuk oder ein Blend davon und das Reaktionsprodukt nach Anspruch 7.

14. Zusammensetzung nach Anspruch 12, wobei die Menge der in der Kautschukzusammensetzung eingesetzten Zersetzungsschutzmittel 0,5 phr bis 5,0 phr beträgt.

15. Zusammensetzung, umfassend natürlichen oder synthetischen Kautschuk oder ein Blend davon und eine Mischung aus zwei oder mehr Zersetzungsschutzmitteln, die aus den Zersetzungsschutzmitteln nach Anspruch 1, 2-Methylthio-p-phenylendiamin und 2-Ethylthio-p-phenylendiamin, ausgewählt sind, oder einem oder mehreren Zersetzungsschutzmitteln, die aus den Zersetzungsschutzmitteln nach Anspruch 1, 2-Methylthio-p-phenylendiamin und 2-Ethylthio-p-phenylendiamin, in Kombination mit einem Nicht-Thio-Zersetzungsschutzmittel ausgewählt sind.

16. Zusammensetzung nach Anspruch 15, wobei das nicht thiosubstituierte Zersetzungsschutzmittel aus der Gruppe ausgewählt ist, die aus Phenylendiaminen, Dihydrochinolinen und Phenolen oder einer Mischung davon besteht.

17. Zusammensetzung nach Anspruch 12, wobei der Kautschuk Polyisopren ist.

18. Zusammensetzung nach Anspruch 12, umfassend 0,1 phr bis 5 phr Schwefel, 0,5 phr bis 2 phr eines Vulkanisationsbeschleunigers, 0,1 phr bis 5 phr der Zersetzungsschutzmittel und eine C₁₂-C₂₀-Fettsäure.

19. Zusammensetzung nach Anspruch 18, wobei der Beschleuniger ein Sulfenamid ist.

20. Zusammensetzung nach Anspruch 18, umfassend 2 phr bis 3 phr der Zersetzungsschutzmittel.

## Revendications

1. Composition comprenant des p-phénylènediamines 2-alkylthio- ou 2-aryl(hétéroyl)thio-substituées ayant la formule: 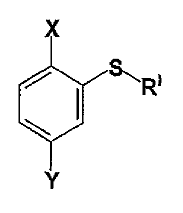 où :
X et Y sont identiques ou différents, et sont choisis dans le groupe NH₂ ou NHR (où R est H, un groupe alkyle, cycloalkyle ou aryle) ; et R' est un groupe alkyle, cycloalkyle, alkylène, aryle, arylène, 3-propionate d'alkyle, des groupes de liaison ayant la formule : - (R" -Z-R") -, où Z est O, NH, NR, S, -SS- ou -(CH₂)ₙCO(R")OC(CH₂)ₙ-, où n = 1 à 3 et R" n' es t pas H et est choisi dans le groupe constitué des groupes alkylène, arylène, pentaérithrityle et hétérocycliques à base d'atomes de carbone contenant au moins un de S ou N, ou S et N, à l'exception de la 2-méthylthio-p-phénylènediamine et de la 2-éthylthio-p-phénylènediamine.

2. Composition selon la revendication 1 comprenant une p-phénylènediamine hétéroylthio-substituée dans laquelle R' est un fragment hétérocyclique choisi dans le groupe constitué des 2-pyrazines, 3-pyrimidines, 2,3,4-pyridines, 2-pyrimidines, 2-(4,6-diméthyl)pyrimid3nes et triazènes substitués.

3. Composition selon la revendication 1 comprenant une p-phénylènediamine hétéroylthio-substituée dans laquelle R' est un fragment hétérocyclique choisi dans le groupe constitué de 1,3,5-triazinyle, 2,5-thiadiazolyle et 2,6-pyridyle.

4. Composition selon la revendication 1, dans laquelle X est une p-phénylènediamine asymétrique ayant un groupe aminoalkyle, Y est un fragment aminoaryle et le groupe p-phénylènediamine 2-alkylthio- ou 2-aryl(hétéroyl)thio substitué est en position 2 par rapport audit groupe aminoalkyle de ladite p-phénylènediamine asymétrique.

5. Composition selon la revendication 1, dans laquelle les groupes alkyle, cycloalkyle, aryle, arylène et alkylène ont de 2 à 18 atomes de carbone.

6. Composition selon la revendication 1, dans laquelle les groupes alkyle, cycloalkyle, aryle, arylène et alkylène ont de 2 à 12 atomes de carbone.

7. Procédé de fabrication de p-phénylènediamines 2-alkylthio- ou 2-aryl(hétéroyl) thio-substituées selon la revendication 1 ou de 2-méthylthio-p-phénylènediamine ou 2-éthylthio-p-phénylènediamine, comprenant la réaction d'une quinone diimine et d'un thiol selon l'équation réactionnelle suivante : 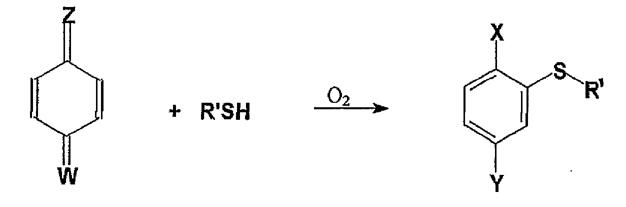 où Z et W sont identiques ou différents, et sont choisis dans le groupe NH ou NR, R et R' étant identiques ou différents et étant choisis parmi les groupes alkyles, cycloalkyles ou aryles ;
où X et Y sont identiques ou différents, et sont choisis dans le groupe NH₂ ou NHR.

8. Procédé selon la revendication 7, dans lequel la quantité de R'SH utilisée dans la réaction est de 10 % à 90 % de la stoechiométrie exigée pour donner un adduit 1:1, donnant un produit réactionnel comprenant un mélange de p-phénylènediamines 2-alkylthio- ou 2-aryl(hétéroyl)thio-substituées et de quinone diimine non réagie.

9. Procédé selon la revendication 7, dans lequel la réaction est conforme à l'équation réactionnelle suivante :

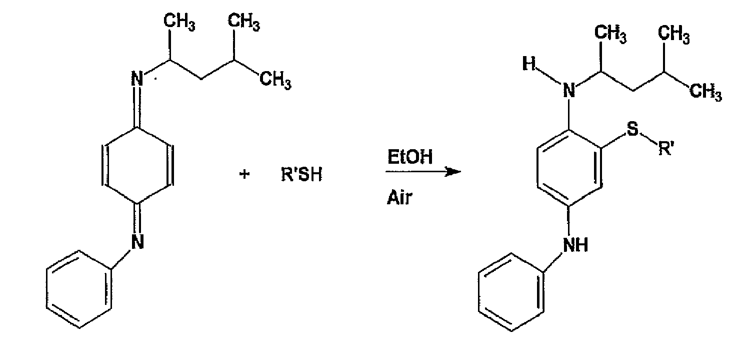

10. Procédé selon la revendication 7, dans lequel les conditions réactionnelles comprennent l'agitation des réactifs dissous dans un solvant approprié pendant au moins 2 heures sous un courant constant d'air à une température de 20 °C à 25 °C.

11. Procédé selon la revendication 10, dans lequel ledit solvant comprend l'éthanol.

12. Composition comprenant un caoutchouc naturel ou synthétique ou un mélange de ceux-ci et un ou plusieurs antidégradants choisis à partir de la composition de la revendication 1, la 2-méthylthio-p-phénylènediamine et la 2-éthylthio-p-phénylènediamine.

13. Composition comprenant un caoutchouc naturel ou synthétique ou un mélange de ceux-ci et le produit réactionnel de la revendication 7.

14. Composition selon la revendication 12, dans laquelle la quantité d'antidégradants utilisée dans la composition de caoutchouc est de 0,5 phr à 5,0 phr.

15. Composition comprenant un caoutchouc naturel ou synthétique ou un mélange de ceux-ci et un mélange de deux antidégradants ou plus choisis parmi les antidégradants de la revendication 1, la 2-méthylthio-p-phénylènediamine et la 2-éthylthio-p-phénylènediamine, ou un ou plusieurs antidégradants choisis parmi les antidégradants de la revendication 1, la 2-méthylthio-p-phénylènediamine et la 2-éthylthlo-p-phénylènediamine en combinaison avec un antidégradant sans groupe thio.

16. Composition selon la revendication 15, dans laquelle ledit antidégradant substitué par un groupe autre qu'un thio est choisi dans le groupe constitué des phénylènediamines, des dihydroquinoléines et des composés phénoliques, ou d'un mélange de ceux-ci.

17. Composition selon la revendication 12, dans laquelle ledit caoutchouc est le polyisoprène.

18. Composition selon la revendication 12, comprenant de 0,1 phr à 5 phr de soufre, de 0,5 phr à 2 phr d'un accélérateur de vulcanisation, de 0,1 phr à 5 phr desdits antidégradants et un acide gras en C₁₂-C₂₀.

19. Composition selon la revendication 18, dans laquelle ledit accélérateur est un sulfénamide.

20. Composition selon la revendication 18, comprenant de 2 phr à 3 phr desdits antidégradants.
